# EUROPÄISCHE PATENTSCHRIFT

(11) **EP 2 575 949 B1**
(45) Veröffentlichungstag und Bekanntmachung des Hinweises auf die Patenterteilung: **06.11.2019**
(21) Anmeldenummer: 11761261.4
(22) Anmeldetag: 01.06.2011
(51) Int. Cl.: A61M 37/00, A61M 39/22, A61M 39/28, F16K 7/04

(54) **ANORDNUNG MIT EINER VENTILVORRICHTUNG ZUM STEUERN EINES FLUSSES EINES FLUIDES DURCH EINEN FLUIDKANAL**
ARRANGEMENT OF A VALVE DEVICE FOR CONTROLLING A FLOW OF A FLUID THROUGH A FLUID CHANNEL
SYSTÈME DE VANNE PERMETTANT DE RÉGULER LE DÉBIT D'UN FLUIDE DANS UN CANAL DE FLUIDE

(30) Priorität: 02.06.2010 DE 102010017216
(43) Veröffentlichungstag der Anmeldung: 10.04.2013
(73) Patentinhaber: Technische Universität Berlin, 10623 Berlin (DE)
(72) Erfinder: LOTH, Andreas, 13156 Berlin (DE); BÜHS, Florian, 10777 Berlin (DE)
(74) Vertreter: Bittner, Thomas L.
(86) Internationale Anmeldenummer: PCT/DE2011/075127
(87) Internationale Veröffentlichungsnummer: WO 2012/022314

(56) Entgegenhaltungen:
- EP-A1- 0 455 478
- WO-A1-2009/149137
- DE-A1- 4 130 601
- US-A- 2 816 514
- US-B1- 7 260 932

## Beschreibung

Die Erfindung betrifft eine Anordnung zum Steuern eines Flusses eines Fluides durch einen Fluidkanal.

### Hintergrund der Erfindung

Derartige Ventilvorrichtungen werden genutzt, um den Volumenstrom eines Fluides durch einen Fluidkanal einzustellen und zu verändern. Es ist in diesem Zusammenhang bekannt, den Fluss des Fluides durch den Kanal dadurch zu steuern, dass von außen auf einen Schlauch, in dem der Fluidkanal gebildet ist, Druck ausgeübt wird, wobei der Druck in Abhängigkeit von einem gewünschten Volumenstrom des Fluides durch den Kanal angepasst wird.

In der Medizintechnik erfolgt die Dosierung von Volumenströmen zum Beispiel über Mikroventile oder direkt über eine entsprechende Pumpe, mit der das abzugebende Fluid mit Druck beaufschlagt wird. Anwendung finden Ventile in der Medizin und im kosmetischen Bereich beispielsweise bei der Injektion von medizinisch und kosmetisch wirksamen Substanzen. Neben der Injektion von Substanzen zur Fettreduktion oder Faltenunterspritzung ist auch beim Farbeintrag für Tätowierungen oder permanentes Make-up im kosmetischen Bereich die dosierte Abgabe einer Substanz notwendig. Medizinische Anwendungen sind neben unterschiedlichen Impfungen auch zum Beispiel die Mesotherapie. Bei den genannten Anwendungen kann neben dem einfachen Eintrag von Medien auch die gleichzeitige Gabe von mehreren Medien vorgesehen sein, so dass diese erst nach dem Eintrag miteinander reagieren.

Das Dosieren des Fluides kann mittels einer berührenden oder einer berührungslosen Verfahren ausgeführt werden. Berührungslose Geräte werden auch als so genannte Dispenser bezeichnet. Der Vorgang der Fluidabgabe selbst wird Dispensen, Jetten oder Pulsen genannt. Dieses Dosieren dient dem Eintrag von Substanzen auf die Haut oder in nachgeschaltete Substanzabgabesysteme (vgl. zum Beispiel EP 1 882 491). Berührend kann als das Dosieren auf eine Oberfläche oder durch diese hindurch verstanden werden.

Allen Anwendungen gemein ist der Bedarf einer präzisen Dosierung. Probleme bereiten oftmals chemische oder fluidische Eigenschaften, Partikel oder wechselnde Medien.

Das Dokument DE 103 37 484 B4 beschreibt ein berührungsloses Dosiersystem, bei dem ein Schlauch mit hoher Geschwindigkeit gequetscht wird, so dass sich ein frei fliegender Flüssigkeitstropfen bildet. Dosierfrequenzen von 50 Hz können auf diese Weise erreicht werden. Es handelt sich bei dem Aufbau um ein offenes System ohne Vordruck. Die Flüssigkeit befüllt den Schlauch aufgrund der Kapillarkräfte. Durch diesen Aufbau ist jedoch die maximale Dosiermenge und Dosierfrequenz beschränkt. Eine Funktion bei Gegendruck ist nicht oder nur sehr begrenzt möglich.

Im Dokument DE 693 25 591 T2 werden Ventilanordnungen zum Schalten einer Strömung durch flexible Schläuche beschrieben. Über einen schwenkbaren Hebel können zwei Positionen (bistabil auf / zu) angewählt werden. An die angekoppelten Flansche dieser mittels Gießen und Schweißen gefertigten Konstruktion sollen die Flüssigkeiten das Ventil durchströmen. Eine mögliche Kontamination der Flüssigkeit wird nicht verhindert, ebenso wenig wie sich das Prinzip nicht als Einwegteil oder für höhere Frequenzen (> 1 Hz) nutzen lässt.

Aus dem Dokument US 3,335,753 ist eine Ventileinrichtung bekannt, bei der mittels eines Quetschelementes der Durchfluss durch einen flexiblen Schlauch einstellbar ist. Das Quetschelement ist auf einem drehbar gelagerten Stellelement angeordnet, welches zum Verlagern des Quetschelementes betätigbar ist, wobei eine Rückholkraft mittels einer Feder bereitgestellt ist.

Das Dokument DE 20 2005 009 350 U1 offenbart eine Schlauchklemme.

Ein Schlauchventil ist aus dem Dokument DE 1 707 336 bekannt.

Im Dokument DE 2 353 624 ist eine Mikrosteuerungsvorrichtung für die Durchströmung von flexiblen Leitungen mit Flüssigkeiten beschrieben. Bei der bekannten Vorrichtung wird die flexible Leitung mit Hilfe eines betätigbaren Hebelarms gequetscht oder freigegeben.

Das Dokument EP 1 699 560 B1 beschreibt eine Möglichkeit, kleinste Mengen zu Pipetieren, basiert jedoch im Wesentlichen auf einer Kombination herkömmlicher Pipetiersysteme und dem bekannten Pipejet-Verfahren, also einer Schlauchdeformation, in diesem Fall als Pipettenspitze ausgeführt. Es ist so lediglich ein Dosieren kleinster Tropfen möglich, die frei fliegend zu ihrem Ziel gelangen. Für Injektionen lässt sich das Verfahren nicht nutzen, da ein Arbeiten bei Gegendruck nicht möglich ist.

Das Dokument DE 197 06 513 C2 beschreibt ein Mikrodosierverfahren, basierend auf einer Druckkammer mit Reservoiranschluss und Fluidauslass. Die Druckkammer wird durch einen Verdränger verkleinert, so dass Fluid zum Auslass gedrückt wird. Wesentlich ist hier eine Einrichtung zum Erfassen der Verdrängerposition.

Das Dokument DE 41 30 601 A1 betrifft ein Hydrocephalusventil zur Erzeugung eines Gegendruckes zur Aufrechterhaltung eines konstanten, von Lage und Liquorfluss unabhängigen Druckes im Schädel von Hydrocephaluspatienten. Der für den Schädel notwendige Druck wird durch ein geregeltes Drosselventil erzeugt, das sich in einer festen Kapsel befindet und das einmal durch eine Feder, zum anderen aber durch einen lageabhängigen Druck belastet wird.

In dem Dokument WO 2009/149137 A1 ist eine piezoelektrisch gesteuerte Ventilvorrichtung offenbart. Ein beweglicher Arm ist mit einer Aktoreinrichtung gekoppelt. Mittels der Aktoreinrichtung wird der bewegliche Arm bewegt, sodass eine am Arm befestigte Klemme einen Druck auf einen Schlauch ausübt.

In dem Dokument US 7,260,932 B1 ist eine Ventileinrichtung mit einer Formgedächtnislegierung beschrieben. Mittels der Formgedächtnislegierung ist ein Arm mit einem Quetschelement zwischen verschiedenen Stellungen verlagerbar.

Das Dokument EP 0 455 478 A1 beschreibt eine Kathetereinrichtung mit einer Ventilvorrichtung.

Aus dem Dokument US 2,816,514 ist eine Fluidpumpe bekannt, bei der in einem Schlauch mit einem zu pumpenden Fluid eine längs des Schlauches sich fortbewegende Pumpkammer erzeugt wird, indem ein auf der Außenfläche des Schlauches angeordneter Streifen in Wellenbewegungen versetzt wird. Dieses geschieht mit Hilfe eines Schwingungsarms, der mit Hilfe eines Aktors in Schwingung versetzt wird und der Schwingungsfrequenz entsprechend an den Streifen auf der Außenfläche des Schlauches koppelt.

### Zusammenfassung der Erfindung

Aufgabe der Erfindung ist es, verbesserte Technologien in Verbindung mit einer Anordnung mit Druckbeauflagungseinrichung und Ventilvorrichtung zum Steuern eines Flusses eines Fluides durch einen Fluidkanal anzugeben, mit denen eine zuverlässige Steuerung des Volumenstroms des Fluides gewährleistet ist, insbesondere auch beim hochfrequenten Betätigen der Ventilvorrichtung.

Diese Aufgabe wird erfindungsgemäß durch eine Anordnung nach dem unabhängigen Anspruch 1 gelöst. Vorteilhafte Ausgestaltungen der Erfindung sind Gegenstand von abhängigen Unteransprüchen.

Die Erfindung umfasst den Gedanken einer Anordnung mit einer Druckbeauflagungseinrichung und einer Ventilvorrichtung zum Steuern eines Flusses eines Fluides durch einen Fluidkanal mit einem Schlauch aus flexiblem Material, in welchem ein Abschnitt eines Fluidkanals gebildet ist, einem dem Schlauch zugeordneten Ventilbauteil, einem Kragarm, der an dem Ventilbauteil gegen eine vom Kragarm wenigstens teilweise selbst bereitgestellte Rückstellkraft reversibel verlagerbar gebildet ist, einem Quetschelement, welches an einem zur Lagerung des Kragarmes an dem Ventilbauteil distalen Ende des Kragarmes angeordnet und konfiguriert ist, mittels Drücken gegen eine Außenfläche des Schlauches einen Fluss eines Fluides durch den Fluidkanal zu steuern, wahlweise bis hin zum Schließen des Fluidkanals, und einem dem Quetschelement zugeordneten Aktor, welcher konfiguriert ist, das Quetschelement gegen die Rückstellkraft am Kragarm in verschiedene Regelstellungen zu verlagern, wodurch der Druck des Quetschelementes auf die Außenfläche des Schlauches und hierdurch den Fluss durch den Fluidkanal regelbar ist.

Nach einem weiteren Aspekt der Erfindung ist eine Anordnung mit zumindest einer Ventilvorrichtung und einer mit dem Fluidkanal in Fließverbindung stehender Druckbeaufschlagungseinrichtung vorgesehen, die konfiguriert ist, das Fluid in dem Fluidkanal mit einem Druck zu beaufschlagen.

Ein weiterer Aspekt der Erfindung betrifft eine Anordnung mit einer Mehrwegeventilvorrichtung mit mehreren Ventilvorrichtungen zum Steuern eines Flusses eines Fluides durch einen Fluidkanal.

Mit der erfindungsgemäßen Anordnung mit einer Ventilvorrichtung ist die Möglichkeit geschaffen, den Volumenstrom durch den im Schlauch gebildeten Fluidkanal jeweiligen Anwendungserfordernissen entsprechend individuell einzustellen und zu regeln, insbesondere auch mit hohen Veränderungs- oder Wiederholfrequenzen. Die mittels des Aktors bewirkte Verlagerung des Quetschelementes und die hierdurch erfolgte Veränderung des Durchflusses durch den Schlauch lässt sich in einer möglichen Anwendung auch hochfrequent mittels entsprechender repetierender Aktorbewegung ausführen. Der Aktor kann dem jeweiligen Anwendungsfall genügend ausgestaltet werden, so dass ein ausreichender Verlagerungsweg für das Quetschelement bereitgestellt ist. So kann beispielsweise das Quetschelement aus einer ersten Stellung, in welcher der Fluidkanal in dem Schlauch vollständig geöffnet ist, in eine zweite Stellung verlagert werden, in welcher der Fluidkanal im Wesentlichen geschlossen ist. Bei dieser Ausgestaltung wird die Ventilvorrichtung in einer Nutzungsart als so genannter Schließer eingesetzt. Auch eine umgekehrte Verlagerung des Quetschelementes kann vorgesehen sein, was dann einer Ausführung als so genannter Öffner entspricht.

Der Kragarm kann ein- oder mehrteilig ausgeführt sein. So können in einer Ausgestaltung mehrere Kragarme vorgesehen sein, die wahlweise zusammen wirken.

Der Aktor kann in einer Ausführungsform direkt auf das Quetschelement einwirken, also direkt hieran koppeln, um dieses zu verlagern. Es kann aber auch vorgesehen sein, dass der Aktor an einem anderen Abschnitt des Kragarmes angreift, also hieran koppelt, um diesen gegen die Rückstellkraft zu schwenken, wodurch das Quetschelement am distalen Ende des Kragarms verlagert wird.

Die Ventilvorrichtung kann in beliebigen Geräten oder Geräteteilen verwendet werden, um den Strom eines Fluides durch den Fluidkanal in einem Schlauch zu steuern. Insbesondere ist eine Verwendung in einer Dosiereinrichtung vorteilhaft, mit der auf dem Gebiet der Medizin oder der Kosmetik ein medizinischer oder kosmetischer Wirkstoff dosiert abzugeben ist. Die Dosiereinrichtung kann in berührender oder nicht berührender Bauart ausgeführt sein. Die Dosiereinrichtung kann zum Beispiel in eine Injektionsvorrichtung integriert sein, um die dosierte Abgabe eines Wirkstoffes bei Gegendruck zu steuern. Bei dieser Ausgestaltung ist die Ventilvorrichtung vorzugsweise einer den Wirkstoff abgebenden Kanüle nachgelagert. Die Kanüle sticht in die Haut ein und kann zum Beispiel bei einer vorgegebenen Stichtiefe den Wirkstoff abgeben. Die Dosiereinrichtung ist also nutzbar bei einem Verfahren zum dosierten Abgeben eines Fluides, insbesondere zum Injizieren in den menschlichen oder tierischen Körper.

Bevorzugt ist das Quetschelement am Kragarm mit einem Vorsprung in Richtung des Schlauches gebildet, der von außen gegen den Schlauch drückt. Eine dem Schlauch zugeordnete Quetschoberfläche des Quetschelementes kann eine beliebige Oberflächenkontur aufweisen, zum Beispiel eine abgerundete Fläche, eine kugelförmige Oberfläche oder eine Oberfläche mit einer Quetschkante, die im Bereich von zwei schräg aufeinander zulaufenden Oberflächen gebildet ist. Auch eine Kombination solcher Oberflächenkonturen kann vorgesehen sein, insbesondere zur Optimierung der Quetschwirkung.

Bei einer zweckmäßigen Ausgestaltung der Erfindung kann vorgesehen sein, dass sich der Kragarm längs des Schlauches erstreckt und mit der Längsrichtung des Schlauches wahlweise einen spitzen Winkel einschließt. In einer Ausführungsform verläuft der Kragarm im Wesentlichen parallel zur Längsrichtung des Schlauches.

Eine vorteilhafte Ausführungsform der Erfindung sieht vor, dass der Aktor mit wenigstens einem Aktorlement ausgewählt aus der Gruppe von Aktorelementen gebildet ist: elektrisches Aktorelement, magnetisches Aktorelement, piezoelektrisches Aktorelement, pneumatisches Aktorelement, mechanisches Aktorelement und hydraulisches Aktorelement.

Bevorzugst sieht eine Fortbildung der Erfindung vor, dass der Aktor mit einem Betätigungselement gebildet ist, welches konfiguriert ist, die Verlagerung des Quetschelementes in die verschiedenen Regelstellungen mittels einer Aktorbewegung entlang einer Bewegungsrichtung zu bewirken, die im Wesentlichen senkrecht zur Längsrichtung des Fluidkanals verläuft.

Bei einer vorteilhaften Ausgestaltung der Erfindung kann vorgesehen sein, dass das Ventilbauteil als ein Mikroventilbauteil gebildet ist. Auf diese Weise ist die Ventilvorrichtung beispielsweise für eine Mikrodosiereinrichtung.

Eine Weiterbildung der Erfindung kann vorsehen, dass das Quetschelement den Schlauch wenigstens teilweise umgreifend gebildet ist.

Eine bevorzugte Weiterbildung der Erfindung sieht vor, dass das Quetschelement mit mehreren Teilquetschelementen gebildet ist, die um den herum Schlauch angeordnet sind. In einer Ausführung sind die mehreren Teilquetschelemente den Schlauch im Wesentlichen vollständig umgreifend gebildet. Eine Ausführungsform sieht vor, dass die Quetschelemente an einer gemeinsamen Basis angeordnet sind. Es kann vorgesehen sein, dass die mehreren Teilquetschelemente gemeinsam mittels eines oder mehrerer Aktoren betätigt werden. Diese Bewegung kann gleichartig und / oder gleichzeitig erfolgen. In einer bevorzugten Ausgestaltung sind die mehreren Teilquetschelemente rotationssymmetrisch um den Schlauch herum gebildet. In einer Fortbildung der Erfindung ist die Anordnung der mehreren Teilquetschelemente im Wesentlichen einem Druckminen-Mechanismus entsprechend ausgeführt.

Bei einer zweckmäßigen Ausgestaltung der Erfindung kann vorgesehen sein, dass die zumindest ein Teil der mehreren Teilquetschelemente wenigstens in einer Endstellung aufeinander lagernd angeordnet sind. Hierbei kann vorgesehen sein, dass in der Endstellung, in welcher mittels Quetschen des Schlauches der Fluidkanal teilweise oder vollständig geschlossen ist, einzelne Teilquetschelemente sich aufeinander abstützen, so dass in einer Ausführungsform ein weiteres Schließen des Fluidkanals nicht mehr möglich ist.

Eine vorteilhafte Ausführungsform der Erfindung sieht vor, dass der Kragarm eine Verlagerung des Quetschelementes zum Öffnen und wenigstens teilweisen Schließen des Fluidkanals im Schlauch mit einer hohen Wiederholfrequenz zulassend ausgelegt ist. Insbesondere sieht eine solche Ausführungsform eine Rückstellkraft vor, die die hochfrequent wiederholte Verlagerung des Quetschelementes unterstützt.

Eine Möglichkeit ist, dass das Ventilbauteil wenigstens zum Teil als ein Spritzgussteil ausgeführt ist. Spritzgussteile sind kostengünstig und auch in Massenfertigung herstellbar. In dieser oder anderen Ausführungsformen kann vorgesehen sein, das Ventilbauteil als Einwegartikel auszuführen. Die Spritzgussausführung des Ventilbauteils kann in einer Weiterbildung in Zwei-Komponenten-Spritzgießen (2K-Spritzgießen) erfolgen.

Bei einer vorteilhaften Ausgestaltung der Erfindung kann vorgesehen sein, dass das Ventilbauteil auf dem Schlauch angeordnet ist. In einer Ausführungsform ist das Ventilbauteil auf dem Schlauch sitzend gebildet, bevorzugt lösbar. Zum Beispiel erfolgt die Anordnung des Ventilbauteils auf den Schlauch mittels einer elastischen Klemmwirkung, die bevorzugt von dem Quetschelement und einem dem Quetschelement zugeordneten Gegenstück bereitgestellt ist. Eine Weiterbildung sieht vor, dass das Ventilbauteil in dieser Ausgestaltung mit einer Schlauchführung gebildet ist, entlang welcher der Schlauch wenigstens abschnittsweise verläuft, wenn das Ventilbauteil auf diesem angeordnet wird. In einer Ausgestaltung verlaufen die Schlauchführung und der Kragarm mit dem Quetschelement im Wesentlichen parallel in Längsrichtung des Schlauches, zum Beispiel oberhalb und unterhalb des Schlauches.

Eine bevorzugte Weiterbildung der Erfindung sieht vor, dass die Anordnung mit einer Ventilvorrichtung ein Einwegmodul aufweist, welches wenigstens mit dem Schlauch und dem Ventilbauteil gebildet ist. Bevorzugt besteht das Einwegmodul aus diesen beiden Elementen.

Bei einer zweckmäßigen Ausgestaltung der Erfindung kann vorgesehen sein, dass der Kragarm an ein Festkörpergelenk koppelt.

Eine vorteilhafte Ausführungsform der Erfindung sieht vor, dass das Festkörpergelenk in einer Hebelanordnung gebildet ist.

Eine vorteilhafte Ausgestaltung der Erfindung kann vorsehen, dass an dem Ventilbauteil wenigstens eine Kupplung gebildet ist, in welche ein Schlauchende mündet. Der Schlauch in Form eines Schlauchstückes bildet mit dem Ventilbauteil ein einheitliches Bauteil, wobei der Schlauch in dem Bauteil verbaut ist, so dass Schlauch und Ventilbauteil als Einheit austauschbar sind. Auch zwei solche Kupplungen können am Ventilbauteil vorgesehen sein, die den beiden Schlauchenden zugeordnet sind. In die Kupplung können Anschlussstücke eingesteckt oder eingeschraubt werden, um die Ventilvorrichtung anzuschließen, zum Beispiel an eine Düse, einen Dispenser, eine Mikromischer, eine Spritze, ein Reservoir oder dergleichen.

In Verbindung mit der Anordnung mit zumindest einer Ventilvorrichtung sowie einer mit dem Fluidkanal in Fließverbindung stehenden Druckbeaufschlagungseinrichtung kann die Integration dieser Anordnung in eine Dosiereinrichtung zum dosierten Abgeben eines Fluides vorgesehen sein, insbesondere eines medizinischen oder kosmetischen Stoffes. Die Fluidabgabe kann hierbei unter Einfluss eines Gegendrucks erfolgen, was insbesondere in Verbindung mit Injektionen von Bedeutung ist. Beispielsweise wird eine solche Injektionseinrichtung in Verbindung mit einer Vorrichtung zum Tätowieren oder zum Ausbilden von permanentem Make-up verwendet. Hierbei wird mit der Ventileinrichtung die Abgabe eines Farbstoffes gesteuert. Aber auch die Dosierung eines anderen kosmetischen Wirkstoffes oder eines medizinischen Stoffes kann vorgesehen sein.

Bei der Mehrwegeventilvorrichtung kann in einer Ausgestaltung vorgesehen sein, dass nebeneinander benachbart angeordnete Fluidkanäle hinsichtlich des sie jeweils durchströmenden Flusses eingestellt werden. Bei einer Ausgestaltung ist ein integriertes Ventilbauteil geschaffen, welches zur Steuerung des Volumenstromes in mehreren Fluidkanälen dient. Zu diesem Zweck verfügt das integrierte Ventilbauteil über mehrere Quetschelemente, die jeweils einem Schlauch mit hierin gebildetem Fluidkanal zugeordnet und mittels eines zugeordneten Aktor verlagerbar sind. In der Mehrwegeventilvorrichtung können auch Kreuzungen zwischen Fluidkanälen ausgebildet sein.

Eine Hintereinanderschaltung mehrerer Ventilbauteile kann geeignet sein, die Dosiergenauigkeit zu erhöhen, insofern als das beispielsweise das Volumen zwischen den Ventilbauteilen ein Fluid unter Druck enthält und durch geschicktes Öffnen und Schließen nur das zwischengespeicherte Volumen abgegeben wird. Beispielsweise kann ein entsprechendes Ventil aus Schläuchen unterschiedlicher Wandstärke oder aus Schlauch Rohrkombinationen bestehen. Der "speichernde" Schlauchabschnitt muss lediglich in der Lage sein, Energie zu speichern die einen Überdruck in diesem Abschnitt aufrechterhält. (Ballon mit einem Ein- und Auslass). Dies ist insbesondere für die rein mechanische Ausführung von Interesse. Die Verschaltung mehrerer Ventilbauteile ist möglich und kann beispielsweise genutzt werden, um Mehrwegeventile darzustellen. Nach dem Ventilbauteil können sich also auch weitere Bauteile anschließen.

Die Ventilvorrichtung kann weiterhin zum An- oder Absaugen verwendet werden. Auch einen Kombination von beidem kann vorgesehen sein, beispielsweise ein Ansaugen und ein Ausstoßen im Wechsel oder mehrstufig hintereinander (beispielsweise: An, An, Aus, Aus, Aus, Aus usw.).

Ein Vorteil der Erfindung ist die Möglichkeit, vordosierte oder wechselnde und oder auskristallisierende / eintrocknende Medien zu dosieren, ohne die Anlage zu beschädigen oder reinigen zu müssen, da der Fluidtransport nur in einem leicht zu wechselnden System stattfindet.

Das Ventilbauteil und die Ventilvorrichtung können zum Beispiel in einem Flüssigkeits-Handling-System oder einem Mikromischer zum Einsatz kommen.

### Beschreibung bevorzugter Ausführungsbeispiele der Erfindung

Die Erfindung wird im Folgenden anhand von bevorzugten Ausführungsbeispielen unter Bezugnahme auf Figuren einer Zeichnung näher erläutert. Hierbei zeigen:
- Fig. 1: eine schematische Darstellung einer Anordnung mit einer Ventilvorrichtung,
- Fig. 2: mehrere Ausgestaltungen für ein Ventilbauteil,
- Fig. 3: eine schematische Darstellung einer Mehrwegeventilvorrichtung, bei der zwei Fluidkanäle parallel zueinander verlaufen,
- Fig. 4: eine schematische Darstellung einer Anordnung mit einer weiteren Ventilvorrichtung,
- Fig. 5: eine schematische Darstellung mit einer Ventilvorrichtung nach einer weiteren Ausgestaltung,
- Fig. 6: eine perspektivische Darstellung einer Ventilvorrichtung in einer weiteren Ausführungsform,
- Fig. 7: eine schematische Darstellung einer weiteren Mehrwegeventileinrichtung,
- Fig. 8: eine schematische Darstellung einer Anordnung mit einer Ventilvorrichtung in Mehrwegeausführung,
- Fig. 9: eine schematische Darstellung eines Ventilbauteils, bei dem das Quetschelement am distalen Ende des Kragarms angeordnet ist,
- Fig. 10: eine in eine Festkörperanordnung integrierte Ventilvorrichtung,
- Fig. 11: eine schematische Darstellung einer Ventilvorrichtung, bei der der Schlauch in einem Ventilkörper angeordnet ist, derart, dass Schlauchenden an Kupplungen koppeln, und
- Fig. 12: eine Schnittdarstellung der Ventilvorrichtung aus Fig. 11.

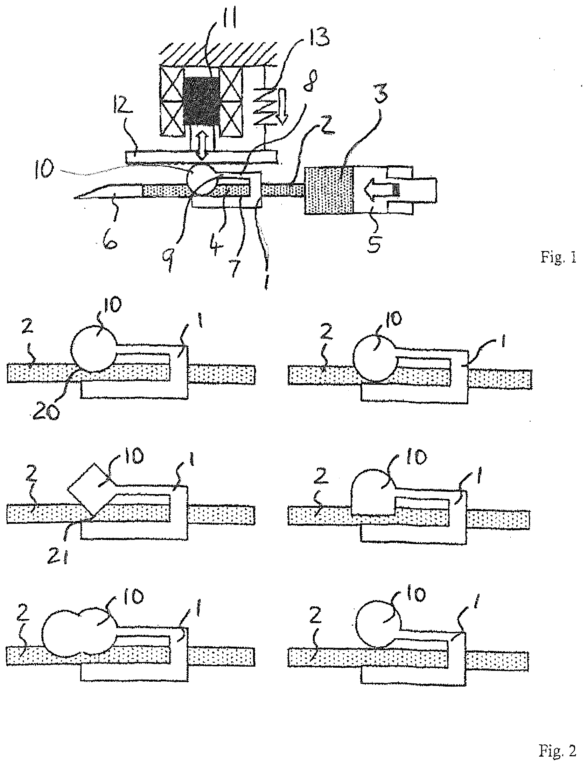

Fig. 1 eine schematische Darstellung einer Anordnung mit einem Ventilbauteil 1, welches auf einem Schlauch 2 angeordnet ist, in dem für ein Fluid 3 ein Fluidkanal 4 gebildet ist, durch welchen das Fluid 3 aus einem mit Druck beaufschlagten Reservoir 5 zu einer Kanüle 6 gelangt, wo das Fluid 3 in dosierter Form abgeben wird. Das Reservoir 5 kann beispielsweise ein Reservoir für Farben sein, die zum Ausbilden eines Tattoos oder von permanentem Make-up dienen.

Das Ventilbauteil 1 ist mit einer Schlauchführung 7 gebildet, auf welcher der Schlauch 2 aufliegt. Der Schlauchführung 7 gegenüberliegend ist ein Kragarm 8 gebildet. Am distalen Ende 9 des Kragarms 8 ist ein Quetschelement 10 angeordnet, welches von außen auf den Schlauch 2 drückt. Das Quetschelement 10 wird entlang einer Bewegungsrichtung verlagert, die im Wesentlichen quer zur Längsrichtung des Schlauches 2 verläuft, indem ein Aktor 11 mit einem Betätigungselement 12 mit dem Quetschelement 10 zusammenwirkt. Bei der Ausführungsform in Fig. 1 wird das Quetschelement 10 gegen eine Rückstellkraft des Kragarmes 8 auf den Schlauch 2 gedrückt, um den Fluss des Fluides durch den Schlauch 2 einzustellen. Die Verlagerung des Betätigungselementes 12 erfolgt bei der dargestellten Ausführungsform weiterhin gegen eine Federkraft 13.

Es sind verschiedene Arten von Aktoren nutzbar, beispielsweise ein pneumatisches, ein hydraulisches, ein elektrisches, ein magnetisches oder ein piezoelektrisches Aktorelement.

Mittels des Quetschelementes 10 lässt sich für ein bekanntes Medium der Volumenstrom einstellen, indem der anliegende Druck, die Öffnungsweite und / oder -zeit geregelt werden. Diese Parameter beeinflussen den Umfang an dosierbaren Substanzen beziehungsweise Viskositäten. Prinzipiell lassen sich alle flüssigen und gasförmigen Substanzen mit im Vergleich zum Schlauchinnendurchmesser nicht zu großen Partikeln dosieren.

Die Betätigung der jeweiligen Klemm- oder Quetschvorrichtung lässt sich rein mechanisch, durch eine Aktor getriebene Betätigung der Klemmvorrichtung erzeugen, die ein Öffnen oder Schließen der mittels des Quetschelementes gebildeten Klemme hervorruft. Die Rückstellung kann insbesondere über die elastische Verformung (Rückstellkraft des Kragarmes 8) und / oder Rückformkräfte des Schlauchs und / oder durch den Druck des im Schlauch befindlichen Mediums auf die Schlauchwand erzielt werden. Auf diese Weise kann ein Dosieren bei hohen Frequenzen erfolgen.

Das Ventilbauteil 1 mit dem Quetschelement 10 kann fest mit dem Schlauch 2 verbunden sein oder an diesem nachträglich befestigt werden. In letzterem Fall kann die Ventilvorrichtung als Mehrwegventil ausgeführt sein. Aufgrund sehr geringer Herstellungskosten, beispielsweise im Spritzgießverfahren aus Kunststoff und der besseren Handhabbarkeit, ist eine Einwegnutzung vorzuziehen, aber auch Varianten aus anderen Materialien wie Metall, Verbundwerkstoffe oder dergleichen sind möglich.

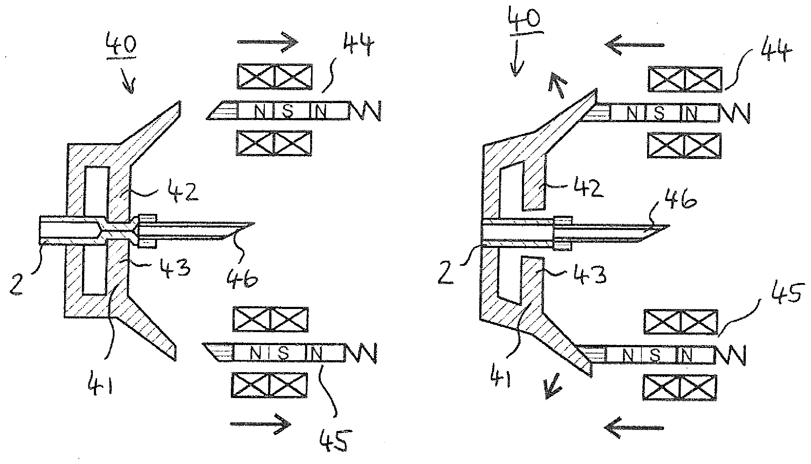

Das Ventilbauteil kann in seinen verschiedenen Ausführungen als Öffner oder Schließer arbeiten. Eine stufenlose Veränderung des Volumenstroms kann gebildet sein. Die Druckbeauschlagung des Schlauches kann ein- oder mehrseitig erfolgen. Unter einseitig wird hier verstanden, dass der Volumenquerschnitt von einer Seite aus begrenzt wird, beispielsweise drückt das Quetschelement von einer Seite auf einen gegenüber dem Quetschelement auf einen Untergrund fixierten Schlauch. Im zweiseitigen Fall ist das Ventilbauteil nach dem Prinzip einer Zange denkbar (vgl. Fig. 4), bei der Klemmbacken gegeneinander geführt werden.

Fig. 2 zeigt mehrere Ausführungsformen für das Ventilbauteil 1, wobei das Quetschelement 10 unterschiedlichen geometrischen Gestaltungen entsprechend gebildet ist. Insbesondere eine Flächenform in einem dem Schlauch 2 zugewandten Quetschabschnitt 20 des Quetschelementes 10 ist auf unterschiedliche Art und Weise gestaltet. Hierzu gehören eine abgerundete Fläche, eine kugelförmige Fläche, eine ebene Fläche sowie ein Flächenbereich mit einer Quetschkante 21.

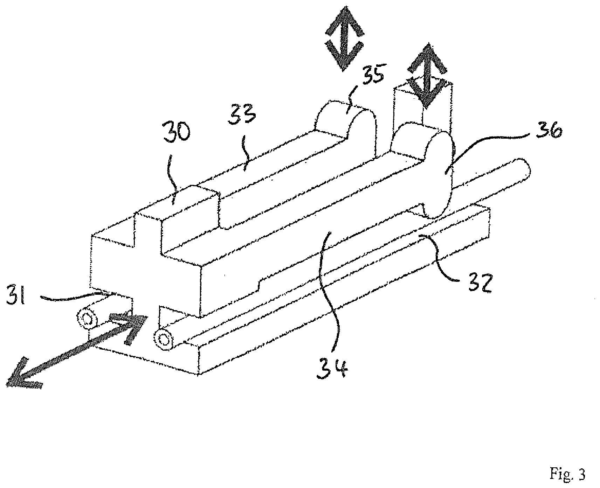

Fig. 3 zeigt eine schematische Darstellung einer Mehrwegeventilvorrichtung, bei der ein Doppelventilbauteil 30 mit parallelen Schlauchführungen 31, 32 gebildet ist. An jeweiligen Kragarmen 33, 34 ist ein zugehöriges Quetschelement 35, 36 gebildet, welches auf den zugeordneten Schlauch von außen drückt.

Fig. 4 zeigt eine schematische Darstellung mit einem Ventilbauteil 40 in einer geschlossenen und einer geöffneten Stellung (vgl. linke und rechte Seite in Fig. 4), bei dem ein Quetschelement 41 mit Klemmbacken 42, 43 gebildet ist, die am zugeordneten Kragarm 8 gebildet sind und die mit Hilfe eines jeweils zugeordneten Aktors 44, 45 betätigt werden. Mittels des Betätigens der Klemmbacken 42, 43 wird der Fluss durch den Schlauch 2 zu einer Kanüle 46 freigegeben oder geschlossen.

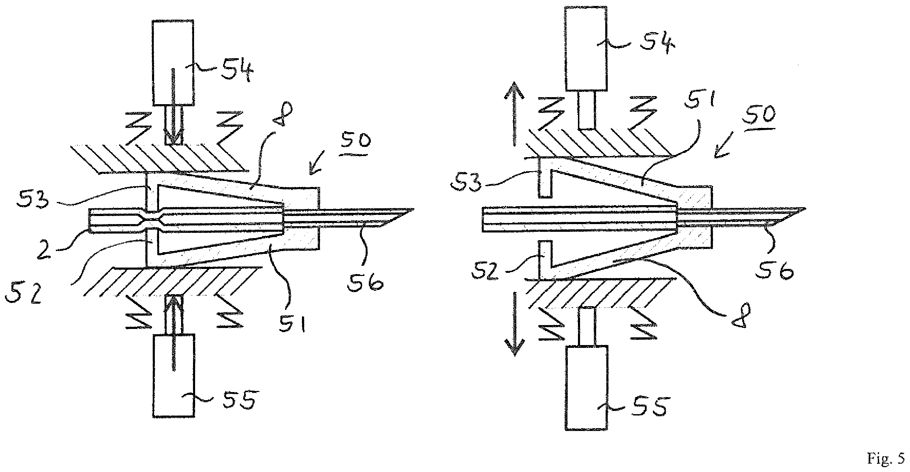

Fig. 5 zeigt eine schematische Darstellung mit einem Ventilbauteil 50, bei dem ein Quetschelement 51 ebenfalls mit zwei Klemmbacken 52, 53 gebildet ist, die am zugeordneten Kragarm 8 gebildet sind und die mit Hilfe zugeordneter Aktoren 54, 55 verlagert werden, um den Fluss durch den Schlauch 2 zu einer Kanüle 56 zu ermöglichen oder zu unterbinden.

Für die in den Fig. 4 und 5 dargestellten Aktoren 42, 43 bzw. 52, 53 können Aktoren mit unterschiedlichen Wirkprinzipien genutzt werden, beispielsweise elektrische, pneumatische, mechanische oder piezoelektrische Aktoren.

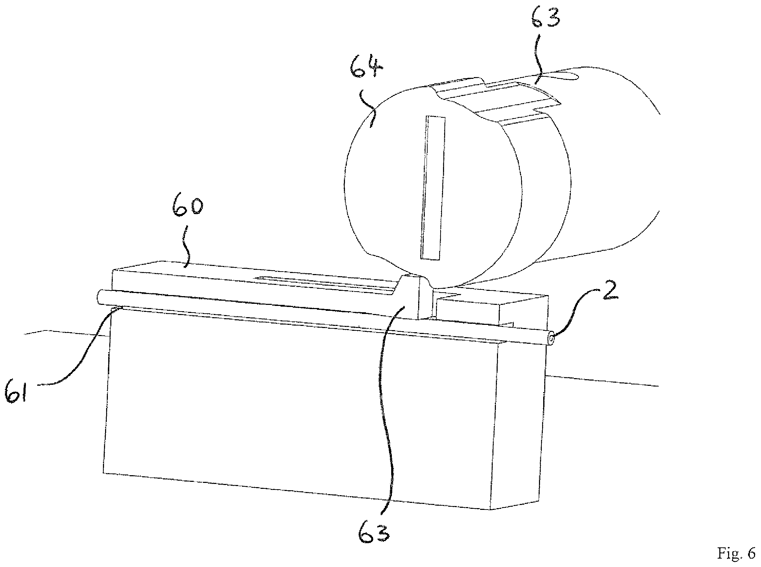

Fig. 6 zeigt eine perspektivische Darstellung einer Ventilvorrichtung in einer weiteren Ausführungsform, bei der ein Ventilbauteil 60 mit einer Führung 61 gebildet ist, in welcher der Schlauch 2 angeordnet ist. Ein zugeordnetes Quetschelement 62, liegt auf der Außenseite des Schlauches 2 und wird mit Hilfe eines mechanischen Aktors 63 repetierend verlagert, indem an dem mechanischen Aktor 63 ein Drehbauteil 64 rotiert. Aufgrund der Oberflächenkontur des Drehbauteils 64 wird das Quetschelement mehr oder weniger gegen den Schlauch 2 gedrückt, wodurch der Fluss durch den Schlauch 2 geregelt wird.

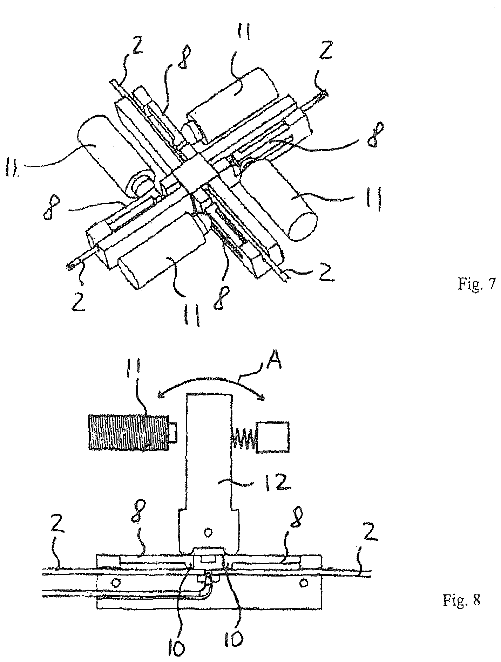

Fig. 7 zeigt eine schematische Darstellung einer Anordnung mit mehreren Ventilen, die bevorzugt jeweils mit einem Ventilbauteil 1 in einer der vorangehend beschriebenen Ausführungsformen sowie einem zugeordneten Aktor 11 gebildet sind. Es ist ein Vier-Wege-Ventil mit Aktoren 11 gebildet.

Fig. 8 zeigt eine schematische Darstellung einer Anordnung mit einer Ventilvorrichtung in Mehrwegeausführung, bei der Quetschelemente 10 wechselseitig betätigt werden, in dem das Betätigungselement 12 des Aktors 11 geschwenkt wird (vgl. Pfeil A in Fig. 8).

In ersten Untersuchungen konnten bereits Abgabefrequenzen von bis zu 400 Hz erreicht werden. Die dosierten Tröpfchen lösten sich dabei sowohl in waagerechter als auch in senkrechter Lage vollständig vom Schlauchende. Als Testflüssigkeit wurde destilliertes Wasser verwendet, bei einem Druck von 2 bar und einem Schlauchdurchmesser von 0,7 X 0,3 mm (außen zu innen). Das saubere Ablösen der einzelnen Tröpfchen wurde mit einer High-Speed-Kamera bei einer Aufzeichnungsfrequenz von 3.500 Hz überprüft. Der Versuchsaufbau wurde bereits im miniaturisierten Maßstab aus Kunststoff hergestellt. Die Abmaße des funktionsfähigen Ventils liegen bei 4x4x15 mm.

Anwendungsbereiche finden sich vor allem in der Injektion von medizinischen und kosmetischen Substanzen. Die Integration in ein Bündel Vollnadeln einer Stech- oder Injektionsvorrichtung kann ebenso vorgesehen sein wie mehrere Kanülen gleichzeitig zu betreiben. Als Beispiele für die kosmetische Behandlungen lassen sich die Carboxytherapie also die Injektion von C02, zum Beispiel zur Fettreduktion, Faltenunterspritzung in Tiefen von 1,0 bis 4,0 mm mit verschiedensten Medien, Tattooeintrag und deren Entfernung (vgl. EP 04 770 455 dort lediglich oberflächliche Absaugung) in Tiefen von 1,0 - 3,5 mm oder Permanent Make-up Eintragung in Tiefen von 0,3 - 1,0 mm nennen. Sowohl zur kosmetischen als auch zur medizinischen Anwendung ist die Absaugung aus der Haut im Allgemeinen denkbar. Rein medizinische Anwendungen sind neben unterschiedlichten Impfungen in Tiefen von 0,2 - 0,6 mm die Mesotherapie in Tiefen von 0,2 - 10 mm.

Die nachfolgende Liste stellt eine Auswahl möglicher Substanzen dar, die eingebracht werden können: Hyaluron-Säure, Vitamin, Q10, Vakzin, therapeutische Antikörper, Krebs-Antikörper, Diabetes-Therapeutikum, Hormon, Zytokine, biochemischer oder biologischer Signalstoff, Anti-Oxidant, Haarwuchsmittel, Haarwachstumshemmer, Mineralstoffe zur Verbesserung der Hautspannung und eines Hautmetabolismus, Enzyme, Co-Enzym, Aminosäure, Nukleinsäure, inerte Farbpartikel, inerter Hautfüller, Nerven aktivierender Wirkstoff wie Botox oder bakterielles Toxin, diabetisches Kontrollmittel wie von der Glycosekonzentration abhängig farbveränderliche Partikel.

Für alle Anwendung ist neben dem einfachen Eintrag von Medien auch die gleichzeitige Gabe von mehreren Medien denkbar, die beispielsweise erst nach der Injektion miteinander reagieren sollen. Ein Teil der Anwendungen hat ein Einbringen oder Entfernen unterschiedlichster Medien in definierter Tiefe beziehungsweise zu einem bestimmten Zeitpunkt gemein. Gerade in der intrakutanen Impftechnik wird deutlich, welchen Stellenwert die genaue Tiefe der Impfung besitzt. So verliert der zu impfende Wirkstoff bereits beim Überschreiten der Zieltiefe um mehr als 15% einen erheblichen Anteil seiner Wirkung.

Neben dem Verpacken kleinster Mengen eines Mediums, wie teuren Medikamenten, lässt sich das gewünschte Mischverhältnis, beispielsweise mit mehreren unterschiedlichen Schlauchdurchmessern, auf einfachste Weise einstellen.

Vor allem in Bereichen wie der Impftechnik sind selbst schwer zu handhabende Reagenzien einfach einsetzbar, da diese in einem geschlossenen Behältnis mit Ventil verwendet werden können und so mit dem eigentlichen Dispenser nicht in Kontakt kommen. Auf ein umständliches Reinigen der Geräte kann daher verzichtet werden.

Es ist weiterhin denkbar, eine Verschlussflüssigkeit, vergleichbar mit einem Korken, beispielsweise eine hydrophile Wirksubstanz und hydrophobe Verschlusssubstanz m verwenden und so ein Auslaufen der eingebrachten Substanz zu verhindern. Auch eine hochviskose Verschlusssubstanz kann zu diesem Zweck verwendet werden. Alternativ lässt sich der Verschluss der Haut durch Koagulieren dieser mit HF, Laser oder anderen Thermoeinwirkungen realisieren.

Aufgrund der einfachen Konstruktion, der vielfältigen Betätigungsvarianten und der geringen Baugröße, lässt sich die Erfindung mit minimalem Aufwand in eine Vielzahl unterschiedlicher Produkte integrieren.

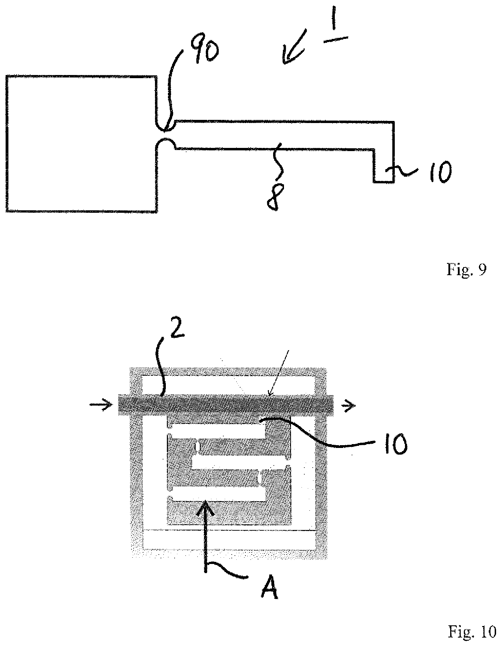

Fig. 9 zeigt eine schematische Darstellung eines Ventilbauteils 1, bei dem das Quetschelement 10 am distalen Ende des Kragarms 8 angeordnet ist und das proximale Ende des Kragarmes 8 an ein Festkörpergelenk 90 koppelt. Das Festkörpergelenk 90 hat den Vorteil, dass hierdurch die zur Ventilbetätigung notwendigen Kräfte minimiert sind. Allgemein handelt es sich bei Festkörpergelenken insbesondere um Bereiche des Kragarms 8, die eine verminderte Steifigkeit aufweisen, sodass dort Gelenk- oder Knickpunkte gebildet sind. Mittels eines Festkörpergelenkes lassen sich deutlich kürzere Hebel beim Kragarm 8 einsetzen, was eine weitere Miniaturisierung der Ventilvorrichtung unterstützt und eine Integration in komplexe Flüssigkeits-Handhabungs-Anordnungen ermöglicht.

Eine weitergehende Miniaturisierung ist dadurch ermöglicht, dass der Schlauch vorgequetscht ist. Wird beispielsweise ein Schlauch mit den Maßen 0,7 x 0,3 mm um 0,2 mm gequetscht, liegt der erreichbare Volumenstrom bei etwa 90% des ungestörten Querschnittes. Dadurch reduziert sich der notwendige Hebelweg von 0,35 mm auf 0,15 mm.

Festkörpergelenke lassen sich leicht während einer Spritzgussfertigung integrieren, indem die Formen entsprechend gestaltet werden. Auch die Fertigung mittels Mikrofräsen oder Erodieren ist bei Metallwerkstoffen möglich.

Die Ventilvorrichtung lässt sich auch direkt in eine Festkörperanordnung integrieren, was beispielhaft in Fig. 10 dargestellt ist. Das Quetschelement 10 liegt auf dem Schlauch 2 auf, welcher von einer Flüssigkeit durchströmt wird. Eine Festgelenkanordnung 100 ist mittels eines Aktors betätigbar, was in Fig. 10 mit dem Pfeil A schematisch dargestellt ist. Die dargestellte Ausführungsform zeigt eine Ausführung einer Kraft-Weg-Übersetzung, bei der am untersten Hebel das Quetschelement 10 gebildet ist. Der in Fig. 10 gezeigte Hebelaufbau mit dem aufgenommenen Schlauch 2 lässt sich beispielsweise kostengünstig mittels Spritzgießen herstellen.

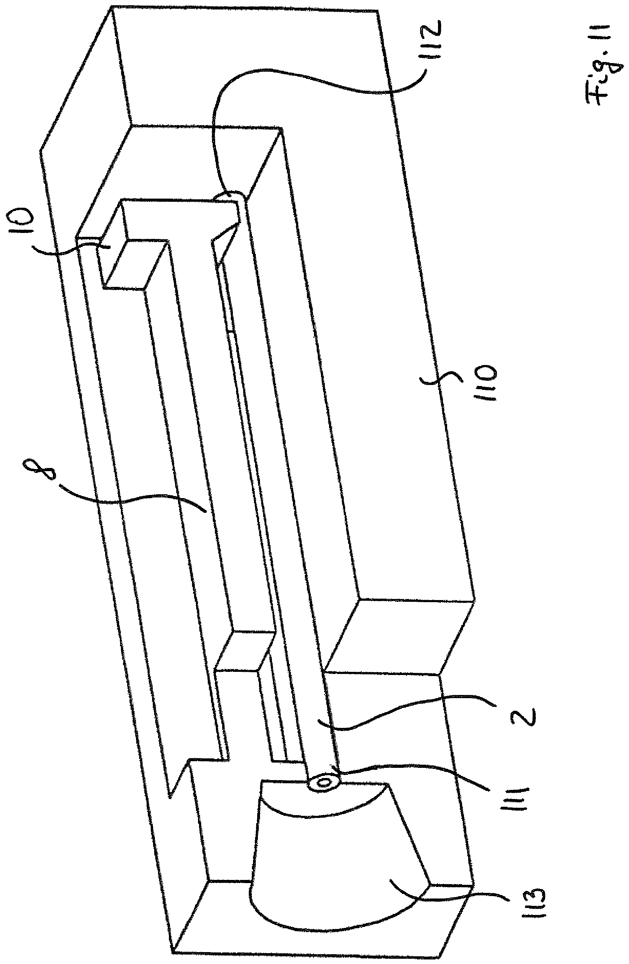

Fig. 11 zeigt eine schematische Darstellung einer Ventilvorrichtung, bei der der Schlauch 2 in Form eines Schlauchstückes in einem Ventilbauteil 110 angeordnet und hierin verbaut ist, derart, dass der Schlauch 2 Schlauchenden 111, 112, die endseitig umlaufend vom Ventilbauteil 110 umgeben sind, in Kupplungen 113, 114 münden, über die die Ventilvorrichtung an Kupplungsstücke oder -anschlüsse (nicht dargestellt) angeschlossen werden kann, die dann vorzugsweise in die Kupplungen 111, 112 eingesteckt oder eingeschraubt werden. Der Kragarm 8 mit dem Quetschelement 10 ist einstückig am Körper des Ventilbauteils 110 gebildet. Die Ventilvorrichtung lässt sich so als Einheit in einem Schritt komplett austauschen, was die Wechselzeit deutlich reduziert und den Gedanken an einen modularen Baukastenansatz verschiedener "Fluidkomponenten" verfolgt.

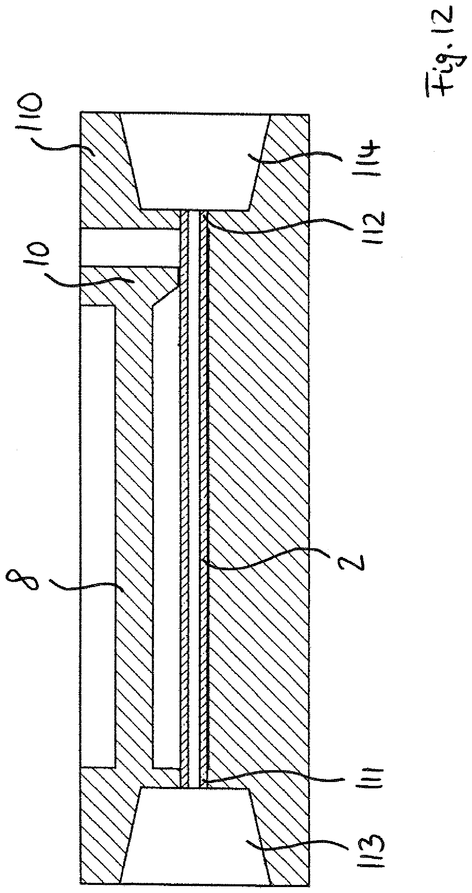

Fig. 12 zeigt eine Schnittdarstellung der Ventilvorrichtung aus Fig. 11.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Anordnung, mit
- einer Ventilvorrichtung, die eingerichtet ist, einen Fluss eines Fluides durch einen Fluidkanal zu steuern, wobei die Ventilvorrichtung als ein Öffner oder ein Schließer ausgeführt ist; und
- einer mit dem Fluidkanal in Fließverbindung stehenden Druckbeaufschlagungseinrichtung, mittels der das Fluid in dem Fluidkanal mit einem Druck beaufschlagbar ist;
**gekennzeichnet durch** die Ventilvorrichtung aufweisend:
- einen Schlauch (2) aus flexiblem Material, in welchem ein Abschnitt eines Fluidkanals gebildet ist;
- einen dem Schlauch (2) zugeordneten Ventilbauteil (1; 40; 50; 60; 110);
- einen Kragarm (8), der an dem Ventilbauteil (1; 40; 50; 60; 110) gebildet ist, wobei der Kragarm (8) gegen eine vom Kragarm (8) wenigstens teilweise selbst bereitgestellte Rückstellkraft reversibel verlagerbar gebildet ist;
- einen Quetschelement (10; 41, 51; 62), welches an einem zur Lagerung des Kragarmes (8) an dem Ventilbauteil (1; 40; 50; 60; 110) distalen Ende des Kragarmes (8) angeordnet ist, wobei das Quetschelement (10; 41, 51; 62) mittels eines Aktors (11; 44, 45;
54, 55) gegen die Rückstellkraft am Kragarm (8) verlagerbar ist und wobei mit dem Quetschelement (10; 41, 51; 62) mittels Drücken gegen eine Außenfläche des Schlauches (2) ein Fluss eines Fluides durch den Fluidkanal steuerbar ist, bis hin zum Schließen des Fluidkanals; und
- der dem Quetschelement (10; 41, 51; 62) zugeordneten Aktor (11; 44, 45; 54, 55), mittels dem das Quetschelement (10; 41; 51; 62) in verschiedene Regelstellungen verlagerbar ist, wodurch der Druck des Quetschelementes (10; 41; 51; 62) auf die Außenfläche des Schlauches (2) und hierdurch den Fluss durch den Fluidkanal regelbar ist.

2. Anordnung nach Anspruch 1, **dadurch gekennzeichnet, dass** sich der Kragarm (8) längs des Schlauches (2) erstreckt und mit der Längsrichtung des Schlauches (2) wahlweise einen spitzen Winkel einschließt.

3. Anordnung nach Anspruch 1 oder 2, **dadurch gekennzeichnet, dass** der Aktor (11; 44, 45; 54, 55) mit wenigstens einem Aktorelement ausgewählt aus der folgenden Gruppe von Aktorelementen gebildet ist: elektrisches Aktorelement, magnetisches Aktorelement, piezoelektrisches Aktorelement, pneumatisches Aktorelement, mechanisches Aktorelement und hydraulisches Aktorelement.

4. Anordnung nach mindestens einem der vorangehenden Ansprüche, **dadurch gekennzeichnet, dass** der Aktor (11; 44, 45; 54, 55) mit einem Betätigungselement (12) gebildet ist, mittels dem die Verlagerung des Quetschelementes (10; 41; 51; 63) in die verschiedenen Regelstellungen mittels einer Aktorbewegung entlang einer Bewegungsrichtung ausführbar ist, die im Wesentlichen senkrecht zur Längsrichtung des Fluidkanals verläuft.

5. Anordnung nach mindestens einem der vorangehenden Ansprüche, **dadurch gekennzeichnet, dass** das Quetschelement (41; 51) den Schlauch (2) wenigstens teilweise umgreifend gebildet ist.

6. Anordnung nach mindestens einem der vorangehenden Ansprüche, **dadurch gekennzeichnet, dass** das Quetschelement (41; 51) mit mehreren Teilquetschelementen (42, 43; 52, 53) gebildet ist, die um den herum Schlauch (2) angeordnet sind.

7. Anordnung nach Anspruch 6, **dadurch gekennzeichnet, dass** die zumindest ein Teil der mehreren Teilquetschelemente wenigstens in einer Endstellung aufeinander lagernd angeordnet sind.

8. Anordnung nach mindestens einem der vorangehenden Ansprüche, **dadurch gekennzeichnet, dass** der Kragarm (8) eine Verlagerung des Quetschelementes (10; 41; 51; 62) zum Öffnen und wenigstens teilweisen Schließen des Fluidkanals im Schlauch (2) mit einer hohen Wiederholfrequenz zulassend ausgelegt ist.

9. Anordnung nach mindestens einem der vorangehenden Ansprüche, **dadurch gekennzeichnet, dass** das Ventilbauteil (1; 40; 50; 60; 110) auf dem Schlauch (2) angeordnet ist.

10. Anordnung nach mindestens einem der vorangehenden Ansprüche, **gekennzeichnet durch** ein Einwegmodul, welches wenigstens mit dem Schlauch (2) und dem Ventilbauteil (1; 40; 50; 60; 110) gebildet ist.

11. Anordnung nach mindestens einem der vorhergehenden Ansprüche, **dadurch gekennzeichnet, dass** der Kragarm (8) an ein Festkörpergelenk koppelt.

12. Anordnung nach Anspruch 11, **dadurch gekennzeichnet, dass** das Festkörpergelenk (19) in einer Hebelanordnung gebildet ist.

13. Anordnung nach mindestens einem der vorhergehenden Ansprüche, **dadurch gekennzeichnet, dass** an dem Ventilbauteil (110) wenigstens eine Kupplung (113; 114) gebildet ist, in welche ein Schlauchende (111; 112) mündet.

14. Anordnung nach mindestens einem der vorhergehenden Ansprüche, **dadurch gekennzeichnet, dass** mehrere Ventilvorrichtungen vorgesehen sind, die der Ventilvorrichtung entsprechend ausgebildet und in einer Mehrwegeventilvorrichtung angeordnet sind.

## Claims

1. A device, with
- a valve arrangement configured to control the flow of a fluid through a fluid channel, wherein the valve arrangement is designed as an opener or a closer; and
- a pressurizing device connected in terms of flow with the fluid channel, which can be used to pressurize the fluid in the fluid channel;
**characterized by** the valve arrangement, comprising:
- a hose (2) made of a flexible material, in which a section of a fluid channel is formed;
- a valve component (1; 40; 50; 60; 110) allocated to the hose;
- a cantilever arm (8) formed on the valve component (1; 40; 50; 60; 110), wherein the cantilever arm (8) is designed so that it can be reversibly displaced against a restoring force at least partially provided by the cantilever arm (8) itself;
- a crimping element (10; 41, 51; 62), which is arranged on a distal end of the cantilever arm (8) for mounting the cantilever arm (8) on a valve component (1; 40; 50; 60; 110), wherein the crimping element (10; 41, 51; 62) can be displaced by means of an actuator (11; 44, 45; 54, 55) against the restoring force on the cantilever arm (8), and wherein the crimping element (10; 41, 51; 62) can be pressed against an outer surface of the hose (2) to control the flow of a fluid through the fluid channel up to closing of the fluid channel; and
- the actuator (11; 44, 45; 54, 55) allocated to the crimping element (10; 41, 51; 62), by means of which the crimping element (10; 41; 51; 62) is displaceable into various regulating positions, wherein the pressure of the crimping element (10; 41; 51; 62) on the outer surface of the hose (2), and thereby the flow through the fluid channel is regulatable.

2. The device according to claim 1, **characterized in that** the cantilever arm (8) extends along the hose (2), and optionally includes an acute angle with the longitudinal direction of the hose (2).

3. The device according to claim 1 or 2, **characterized in that** the actuator (11; 44, 45; 54, 55) is formed with at least one actuator element selected from the following group of actuator elements: electric actuator element, magnetic actuator element, piezoelectric actuator element, pneumatic actuator element, mechanical actuator element and hydraulic actuator element.

4. The device according to one of the preceding claims, **characterized in that** the actuator (11; 44, 45; 54, 55) is formed with an actuating element (12), by means of which the crimping element (10; 41; 51; 63) is displaceable into the various regulating positions by having the actuator move along a direction of movement that runs essentially perpendicular to the longitudinal direction of the fluid channel.

5. The device according to one of the preceding claims, **characterized in that** the crimping element (41; 51) is designed to at least partially encompass the hose (2).

6. The device according to one of the preceding claims, **characterized in that** the crimping element (41; 51) is formed with several partial crimping elements (42, 43; 52, 53), which are arranged around the hose (2).

7. The device according to claim 6, **characterized in that** at least a portion of the several partial crimping elements are arranged one on top of the other at least in an end position.

8. The device according to one of the preceding claims, **characterized in that** the cantilever arm (8) is designed to allow a displacement of the crimping element (10; 41; 51; 62) for opening and at least partially closing the fluid channel in the hose (2) with a high frequency of repetition.

9. The device according to one of the preceding claims, **characterized in that** the valve component (1; 40; 50; 60; 110) is arranged on the hose (2).

10. The device according to one of the preceding claims, **characterized by** a one-way module, which is formed at least with the hose (2) and valve component (1; 40; 50; 60; 110).

11. The device according to one of the preceding claims, **characterized in that** the cantilever arm (8) is coupled to a flexure bearing.

12. The device according to claim 11, **characterized in that** the flexure bearing (19) is formed in a lever assembly.

13. The device according to one of the preceding claims, **characterized in that** the valve component (110) has formed on it at least one coupling (113; 114), into which a hose end (111; 112) leads.

14. The device according to one of the preceding claims, **characterized in that** several valve arrangements are provided, which are designed corresponding to the valve arrangement, and arranged in a multi-way valve arrangement.

## Revendications

1. Agencement avec
- un dispositif de vanne étudié pour guider un écoulement d'un fluide à travers un canal de fluide, dans lequel le dispositif de vanne est réalisé à ouverture ou à fermeture ; et
- un dispositif d'application de pression, en communication d'écoulement avec le canal de fluide, au moyen duquel il est possible d'appliquer une pression au fluide dans le canal de fluide ;
**caractérisé en ce que** le dispositif de vanne présente :
- un tuyau (2) en un matériau flexible dans lequel un tronçon d'un canal de fluide est formé ;
- un élément de vanne (1 ; 40 ; 50 ; 60 ; 110) associé au tuyau (2) ;
- un bras en porte-à-faux (8) formé sur l'élément de vanne (1 ; 40 ; 50 ; 60 ; 110), dans lequel le bras en porte-à-faux (8) est réalisé de manière déplaçable de façon réversible à l'encontre d'une force de rappel mise à disposition au moins partiellement par le bras en porte-à-faux (8) lui-même ;
- un élément de serrage (10 ; 41 ; 51 ; 62) disposé sur une extrémité distale du bras en porte-à-faux (8) pour le montage du bras en porte-à-faux (8) sur l'élément de vanne (1 ; 40 ; 50 ; 60 ; 110), dans lequel l'élément de serrage (10 ; 41 ; 51 ; 62) est déplaçable au moyen d'un actionneur (11 ; 44 ; 45 ; 54 ; 55) à l'encontre de la force de rappel au niveau du bras en porte-à-faux (8) et dans lequel, avec l'élément de serrage (10 ; 41 ; 51 ; 62), en poussant contre une surface extérieure du tuyau (2), il est possible de guider un écoulement d'un fluide à travers le canal de fluide jusqu'à la fermeture du canal de fluide ; et
- l'actionneur (11 ; 44 ; 45 ; 54 ; 55) associé à l'élément de serrage (10 ; 41 ; 51 ; 62) au moyen duquel l'élément de serrage (10 ; 41 ; 51 ; 62) est déplaçable dans différentes positions de réglage, moyennant quoi il est possible de régler la pression de l'élément de serrage (10 ; 41 ; 51 ; 62) sur la surface extérieure du tuyau (2) et de ce fait, l'écoulement à travers le canal de fluide.

2. Agencement selon la revendication 1, **caractérisé en ce que** le bras en porte-à-faux (8) s'étend le long du tuyau (2) et forme, au choix, un angle aigu avec la direction longitudinale du tuyau (2).

3. Agencement selon la revendication 1 ou 2, **caractérisé en ce que** l'actionneur (11 ; 44 ; 45 ; 54 ; 55) est formé avec au moins un élément d'actionnement sélectionné dans le groupe suivant d'éléments d'actionnement : un élément d'actionnement électrique, un élément d'actionnement magnétique, un élément d'actionnement piézoélectrique, un élément d'actionnement pneumatique, un élément d'actionnement mécanique et un élément d'actionnement hydraulique.

4. Agencement selon l'une au moins des revendications précédentes, **caractérisé en ce que** l'actionneur (11 ; 44 ; 45 ; 54 ; 55) est formé avec un élément de manœuvre (12) au moyen duquel il est possible d'exécuter le déplacement de l'élément de serrage (10 ; 41 ; 51 ; 63) dans les différentes positions de réglage au moyen d'un mouvement d'actionneur le long d'une direction de déplacement qui s'étend sensiblement perpendiculaire à la direction longitudinale du canal de fluide.

5. Agencement selon l'une au moins des revendications précédentes, **caractérisé en ce que** l'élément de serrage (41 ; 51) est réalisé de manière à entourer au moins partiellement le tuyau (2).

6. Agencement selon l'une au moins des revendications précédentes, **caractérisé en ce que** l'élément de serrage (41 ; 51) est formé avec plusieurs éléments de serrage partiels (42, 43 ; 52, 53) qui sont disposés tout autour du tuyau (2).

7. Agencement selon la revendication 6, **caractérisé en ce qu'**au moins une partie des plusieurs éléments de serrage partiels sont disposés de manière à reposer les uns sur les autres au moins dans une position finale.

8. Agencement selon l'une au moins des revendications précédentes, **caractérisé en ce que** le bras en porte-à-faux (8) est conçu de manière à autoriser un déplacement de l'élément de serrage (10 ; 41 ; 51 ; 62) pour ouvrir et au moins partiellement fermer le canal de fluide dans le tuyau (2) avec une fréquence de répétition élevée.

9. Agencement selon l'une au moins des revendications précédentes, **caractérisé en ce que** l'élément de vanne (1 ; 40 ; 50 ; 60 ; 110) est disposé sur le tuyau (2).

10. Agencement selon l'une au moins des revendications précédentes, **caractérisé par** un module à usage unique qui est formé au moins par le tuyau (2) et l'élément de vanne (1 ; 40 ; 50 ; 60 ; 110).

11. Agencement selon l'une au moins des revendications précédentes, **caractérisé en ce que** le bras en porte-à-faux (8) s'accouple avec une articulation monolithique.

12. Agencement selon la revendication 11, **caractérisé en ce que** l'articulation monolithique (19) est formée dans un agencement de levier.

13. Agencement selon l'une au moins des revendications précédentes, **caractérisé en ce qu'**au moins un raccord (113 ; 114) est formé sur l'élément de vanne (110), dans lequel débouche une extrémité de tuyau (111 ; 112).

14. Agencement selon l'une au moins des revendications précédentes, **caractérisé en ce que** plusieurs dispositifs de vanne sont prévus, lesquels sont réalisés de manière correspondante au dispositif de vanne et sont disposés dans un dispositif de vanne à plusieurs voies.
